# EUROPEAN PATENT SPECIFICATION

(11) **EP 1 694 684 B1**
(45) Date of publication and mention of the grant of the patent: **03.08.2011**
(21) Application number: 04800503.7
(22) Date of filing: 01.11.2004
(51) Int. Cl.: C07F 7/10

(54) **PROCESS FOR MAKING CAMPTOTHECIN DERIVATIVES**
VERFAHREN ZUR HERSTELLUNG VON CAMPTOTHECIN-DERIVATEN
PROCEDE DE PREPARATION DE DERIVES DE CAMPTOTHECINE

(30) Priority: 17.12.2003 US 530153 P
(43) Date of publication of application: 30.08.2006
(73) Proprietor: BIONUMERIK PHARMACEUTICALS, INC., San Antonio, Texas 78229 (US)
(72) Inventor: WU, Ye, Helotes, TX 78023 (US); NARKUNAN, Kesavaram, San Antonio, TX 78229 (US); WANG, Jianyan, Helotes, TX 78023 (US); KOCHAT, Harry, San Antonio, TX 78248-2208 (US)
(74) Representative: Lucas, Phillip Brian
(86) International application number: PCT/US2004/036192
(87) International publication number: WO 2005/061517

(56) References cited:
- US-B1- 6 194 579
- US-B1- 6 723 849

## Description

### FIELD OF THE INVENTION

This invention relates to a process for making certain camptothecin derivatives and will have application to a semi-synthetic process for making large quantities of highly lipophilic camptothecins that include one or more silicon atoms in the structure.

### BACKGROUND OF THE INVENTION

Highly lipophilic camptothecin derivatives (HLCDs), particularly those containing silicon-based moieties, are effective anticancer drugs. One of the most noted of the silicon-containing HLCDs has the IUPAC name (4S)-4-ethyl-4-hydroxy-11-[2-(trimethylsilyl)ethyl]-1H-pyrano[3':4':6,7]indolizino[1,2-b]quinoline-3,14(4H,12H)-dione, and has also referred to as 7-(2'-trimethylsilyl)ethyl camptothecin (also known as Karenitecin^{™} and BNP1350), currently in human clinical trials in the United States and internationally. United States Patent 5, 910,491 and others describe the compositions, formulations, and processes for making Karenitecin^{™} and other related HLCDs.

Currently known most preferred processes for making Karenitecin^{™} are described and claimed in United States Patent 6,194,579 (the '579 patent), and in United States Patent Application 10/627,444, filed July 25, 2003 (US Patent No. 6723849). In the '579 patent, Karenitecin^{™} and other silicon-containing HLCDs are manufactured by reacting camptothecin with a TMS-aldehyde and a strong oxidizing agent (hydrogen peroxide is preferred) in the presence of a metal sulfate to effect a Minisci-type alkylation. As described in the '579 patent, the resulting alkylation moiety contained one less carbon atom than the TMS-aldehyde, a typical characteristic of the Minisci alkylation.

The prior patented process for synthesizing Karenitecin was efficient in small-scale (lab-scale) production, but improvements were necessary to enable efficient larger scale production. Improvements were needed primarily to boost yields optimizing process parameters and reagents of choice (and accordingly reduce impurities) and also improvement in analytical methods to address impurity profile of the active pharmaceutical ingredient (powder for BNP1350), and to simplify the purification process to make it user friendly and robust for manufacturing scale. The prior process resulted in 50%-60% crude theoretical yield and 25% to 35% isolated yield after column chromatography. The new process demonstrated approximately 80% crude theoretical yield and 45% to 50% isolated yield after double crystallization.

Other prior processes for synthesizing HLCDs can be found in United States Patents 6,150,343 and others. These prior processes utilize a total synthesis route to synthesize the camptothecin skeleton. Due to the relatively low yields and higher costs of these methods when compared to semisynthetic methods, they are considered impractical and inefficient for conducting large-scale synthetic operations.

United States Patent No. 6723849, referred to above, describes and claims a modified process for synthesizing HLCDs. In the modified process disclosed in this patent, the main difference in the process was the addition of a nonpolar, aprotic solvent to the initial mixture of the trimethylsilyl aldehyde reactant in order to boost yields.

### SUMMARY OF THE INVENTION

The synthetic process of this invention is adapted to produce HLCDs having the following structure I: 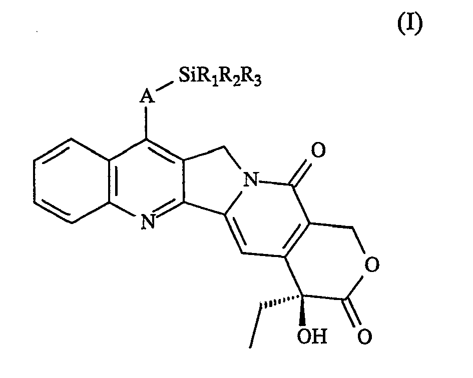 wherein A is -(CH₂)ₘ- where m is 1 to 6;
and R₁, R₂, and R₃ are individually lower alkyl or aryl.

The process of the invention is defined in the claims.

The process is essentially a one-step process for synthesizing the preferred compounds from camptothecin. As is well known, camptothecin can be isolated from the bark of the *camptotheca accuminata* tree, which grows primarily in Asia and parts of Europe. The active form of camptothecin is the (S)-stereoisomer shown above, which can be purchased as a commercial product either in GMP grade or non-GMP grade with substantial purity from any of a number of commercial sources located primarily in China, India and Europe.

In the process of this invention, a modified Minisci alkylation is utilized to synthesize the formula I compound from unsubstituted camptothecin. Instead of a trimethylsilyl aldehyde as initial reactant, the process of this invention utilizes a trialkylsilyl-alkyl- cyclic compound as one of the key intermediates. The process of this invention boosts yields beyond those realized by the previous semisynthetic processes, and is efficient and economical in both small and large-scale process operations.

### DETAILED DESCRIPTION OF THE INVENTION

The preferred embodiments depicted below are not intended to be exhaustive or to limit the invention to the precise forms disclosed. They have been chosen and described to explain the principles of the invention, and its application and practical use to thereby enable others skilled in the art to understand its teachings.

In this application, the term "lower alkyl" means a straight or branched chain hydrocarbon having from one to six total carbon atoms. "Lower alkylene" means a bridging hydrocarbon having from one to six total carbon atoms bonded at its terminal carbons to two other atoms (-CH₂-)ₓ where x is 1 to 6. "Lower alcohol" likewise means an alcohol having from 1 to 6 total carbon atoms. "Aryl means an aromatic ring system, fused or unfused, preferably from one to three total rings, with the ring elements consisting entirely of carbon atoms.

Examples of defined terms include, but are not limited to:
Lower alkyl- methyl, ethyl, propyl, isopropyl, butyl, isobutyl, tert-butyl, etc.
Lower alkylene- methylene, ethylene, propylene, isopropylene, butylene, etc.
Lower alcohol- methanol, ethanol, isopropanol, tert-butyl alcohol, etc.
Aryl- benzyl, phenyl, naphthyl, fluorenyl, and substituted derivatives, etc.

The process of this invention is employed to synthesize compounds of formula I, shown above. Preferred compounds synthesized by the process include those compounds where m is 1, 2 or 3, and R₁, R₂ and R₃ are methyl, *tert*-butyl or phenyl. The process is depicted in the following Schemes.

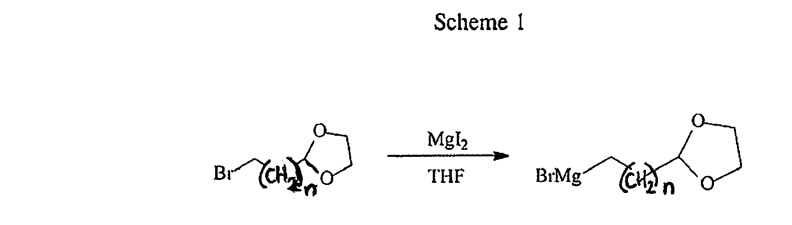

Scheme 1 illustrates the synthesis of an intermediate Grignard reagent used in forming the intermediate that will be reacted with camptothecin to synthesize the formula I compound. As shown in the illustration above, a bromoalkylene heterocyclic compound (illustrated, without limitation as 2-bromoethyl-1,3-dioxolane) is reacted in Grignard fashion with magnesium iodide to form the Grignard reagent shown.

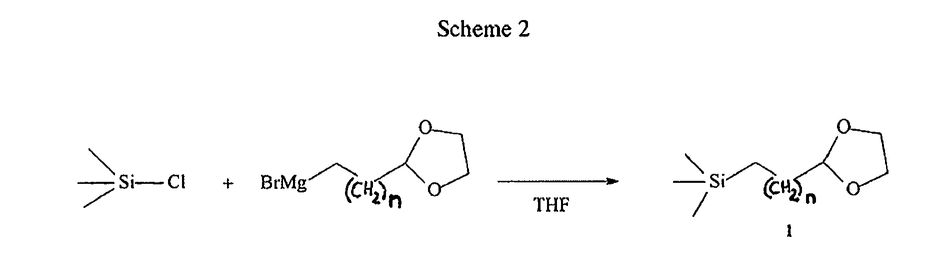

Scheme 2 illustrates the preparation of the silylated reactant from the corresponding Grignard reagent from Scheme 1. In the Scheme, n is 0 to 5 and the alkylene chain linking the terminal silane to the heterocycle may be straight-chain or branched-chain, as desired. Preferably, n is 1 to 3, most preferably 1, and the most preferred end product is 2'-trimethylsilyl-1,3-dioxolane **1.**

The process shown in Schemes 1 and 2 is preferably a one-step, single pot process. All reagents are generally available from commercial sources. As shown, chlorotrimethylsilane is reacted with the Grignard reagent, from Scheme 1, preferably in a nonpolar, aprotic solvent such as tetrahydrofuran (THF).

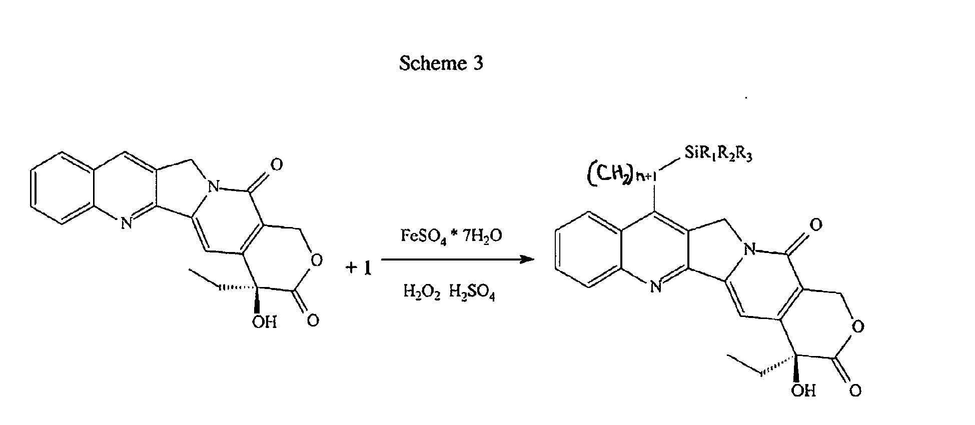

Scheme 3 illustrates the conversion of (S)-camptothecin to the desired formula I compound. The conversion is preferably achieved through the modified Minisci-type hemolytic alkylation reaction.

In the process of this invention, as depicted in Scheme 3, the modified Minisci-type alkylation provides for dissolving the heterocyclic intermediate 1 in a suitable solvent, preferably a low molecular weight pharmaceutically relevant solvents as suitable co-solvent. This solution is then added to a solution of camptothecin in a strong acid capable of protonating the N-1 moiety of camptothecin and a metal sulfate. In prior processes to synthesize the formula I compounds, the reactants tended to be unstable and resulted in low yields partially due to the in situ generation of undesired by-product derived from the co-solvent.

After adding the heterocyclic intermediate **1** to the camptothecin solution, a strong oxidizing agent, preferably hydrogen peroxide is slowly added to the mixture. The formula I compound is then isolated, filtered, washed and purified by recrystallization.

The following specific examples illustrate the process, but are not to be considered as limiting the invention to the precise reagents, steps or conditions depicted.

### Example 1

### Synthesis of 2-(2-trimethylsilylethyl)-1,3-dioxolane

### Equipment Preparation:

Jacketed glass reactor equipped with reflux condenser, overhead stirrer and dropping funnel was dressed up avoiding any residual moisture. The glass reactor was purged with a stream of nitrogen prior to process.

### Reagents and Other Chemicals Used:

| | | |
|---|---|---|
| Anhydrous Tetrahydrofuran | = | 400 mL |
| Magnesium Granules | = | 10 grams (0.411 mol) |
| Trimethylsilyl chloride | = | 36.8 mL (0.290 mol) |
| 2-Bromoethyl-1, 3-dioxolane | = | 50 grams (0.276 mol) |
| Iodine Crystals | = | 100 mg (catalytic) |
| Methyl t-butyl ether | = | 300 mL |
| Anhydrous Sodium sulfate | = | 40 g |

### Process operation:

The reactor was charged with 400 mL of commercially available anhydrous tetrahydrofuran (THF) followed by 10 grams of magnesium granules to form a suspension of magnesium granules in anhydrous THF. To the above suspension was then introduced approximately 100 mg of iodine crystals. The completion of Grignard Reagent formation was visually inspected by insuring complete decoloration of iodine in the reaction medium. The contents were cooled to 0 to 5 °C using cold-water circulation. Once the reaction medium attained the desired temperature, 2-Bromoethyl-1, 3-dioxolane was charged as a thin stream using an overhead-dropping funnel. The reaction mixture was then agitated for approximately 2 hours while allowing the reaction temperature to slowly rise to ambient temperature. The reaction mixture was then cooled back to 0 °C to 5 °C and then charged trimethylsilyl chloride as a thin stream using the overhead-dropping funnel. The reaction mixture was allowed to attain ambient temperature and agitated overnight. Volatiles were then distilled off directly from the reactor. The organic residue was then dissolved in 200 mL methyl t-butyl ether (MTBE) and filter to get rid of magnesium bromide chloride and the excess magnesium granules. The reactor was washed down using another 100 mL of MTBE. The combined organic portion was then charged back to the reactor, washed with 80 mL of process water, organic portion was dried over anhydrous sodium sulfate (40 grams), filtered and concentrated to get the desired crude product (30 grams). The crude product was then distilled between 65- 67 °C at 30 mm Hg reduced pressure to furnish 24.13 grams as colorless oil.
¹H NMR (300 MHz, CDCl₃) δ: -0.001 (9H, s), 0.56-0.62 (2H, m), 1.59-1.67 (2H, m), 3.84-4.00 (4H, m), 4.82 (1H, t, J = 4.7 Hz).

### Example 2

### Synthesis of 7-(2-trimethylsilylethyl) camptothecin (BNP1350)

### Equipment Preparation:

Jacketed glass reactor equipped with reflux condenser, overhead stirrer and dropping funnel was dressed up.

| Reagents and Other Chemicals Used: | | |
|---|---|---|
| Crystallized 20S-Camptothecin | = | 1.3 g (3.73 mM) |
| 2-(2-trimethylsilylethyl)-1,3-dioxolane | = | 1.56 g (8.95 mM) |
| 30% Hydrogen peroxide | = | 1.04 mL |
| Ferrous sulfate heptahydrate | = | 1.3 g |
| t-Butyl alcohol | = | 26 mL |
| 30% Sulfuric acid | = | 78 mL (Stock solution) |
| Cyclohexane | = | 130 mL |
| Dichloromethane | = | 130 mL |
| Anhydrous Sodium sulfate | = | 20 g |
| N,N-dimethyl formamide | = | 22 mL |
| Sodium sulfite | = | 1.04 g |

### Process operation:

The reaction vessel was charged with 78 mL of 30% sulfuric acid. While agitating continued, added 1.3 g of crystallized 20S- camptothecin and 1.3 g of ferrous sulfate heptahydrate at ambient temperature. Continue agitation until both the components were completely dissolved in aqueous sulfuric acid. To the above pale yellow/clear solution was charged as a thin stream using the overhead dropping funnel 1.56 g of 2-(2-trimethylsilylethyl)-1,3-dioxolane dissolved in 26 mL of t-butyl alcohol. After the addition was completed, cooled the reaction medium to 15 °C using the chill water circulation. Once the reaction temperature was at 15 °C, started the drop-wise to a thin stream addition of 1.04 mL of 30% hydrogen peroxide solution through the dropping funnel maintaining the reaction temperature between 15 °C-20 °C. The reaction mixture was agitated for an additional 30 minutes at the same temperature. At the end of 30 minutes, quenched the excess amount of hydrogen peroxide by adding 1.04g of sodium sulfite. An in-process HPLC assay was done at this point to verify the progression of the reaction and the level of impurities formed.

The reactor containing the reaction mixture was charged with 130 mL of Cyclohexane and the contents agitated for 20 minutes. The organic layer was allowed to separate. The cyclohexane portion was drained to a waste container. The reactor was recharged with the aqueous layer. To the above aqueous reaction mixture was added 19.5 mL of cold process water to dilute the aqueous portion. The aqueous layer was then twice extracted with 65 mL of dichloromethane at each time with an agitation time of 15 minutes and standing time of 5 minutes. The aqueous layer was then into a waste container. The combined dichloromethane layer was then recharged into the reactor. The organic portion was washed with 26 mL of process water by agitation for 15 minutes. The water washing was drained into a waste container. The reactor was then charged with 20 g of anhydrous sodium sulfate. The resulting suspension was agitated for 15 minutes. The suspension was filtered through a 10-gram silica gel bed (60 to 100 micron size) to remove sodium sulfate. The pale yellow organic layer thus obtained was then concentrated to obtain light brownish crude product (1.25 g; 74.7% crude yield; HPLC purity = 94.8%).

The crude product was then suspended in 19.5 mL of ethyl alcohol and stirred for 10 minutes. The solid was filtered through a sintered funnel, washed once with 2.6 mL of ethyl alcohol, dried at 40 °C overnight under reduced pressure. The product was analyzed by HPLC and was found 96.9% pure by peak area and weighed to 1.06 g. The product thus obtained was further purified by recrystallizing from 11 mL of anhydrous N,N-dimethyl formamide. The product obtained after crystallization (0.928 g; 55.40% yield) was found 98.9% pure. The DMF recrystallization was repeated one more time to obtain 0.835 g (49.91 % overall yield) of the desired product (BNP1350) with 99.12% purity.
¹H NMR (300 MHz, CDCl₃) δ: 0.18 (9H, s), 0.90-0.96 (2H, m), 1.04 (3H, t, J = 7.4 Hz), 1.82-1.96 (2H, m), 3.08-3.14 (2H, m), 5.24(2H, s), 5.33(1H, d, J = 16.5 Hz), 5.76 (1H, d, J = 16.5 Hz), 7.64-7.69 (2H, m), 7.80 (1H, t, J = 7.2 Hz), 8.04 (1H, d, J = 8.4 Hz), 8.23 (1H, d, J = 8.4 Hz); ¹³C NMR (75 MHz, CDCl₃) δ: -1.63, 8.08, 17.99, 24.35, 31.80, 49.47, 66.54, 72.99, 98.35, 118.68, 123.45, 126.24, 126.75, 127.84, 130.26, 130.77, 147.11, 147.39, 149.47, 150.31, 151.96, 157.82,174.07.

The above description and examples are not limitative of the invention, which is defined by the scope of the following claims.

## Claims

1. A process for synthesizing camptothecin derivatives having the formula: 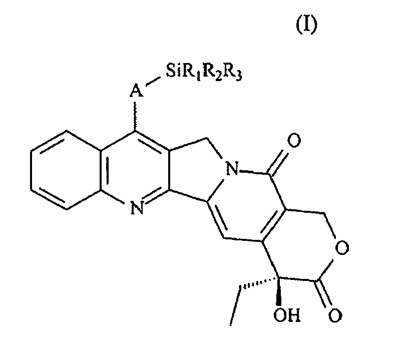 where A is -(CH₂)ₘ-;
m is 1 to 6; and R₁, R₂, and R₃ are individually C₁-C₆ alkyl or aryl; the process comprising:
a) providing a quantity of substantially pure camptothecin, dissolving said camptothecin in a solution of a strong acid, and adding a quantity of a metal hydrate catalyst to the strong acid solution;
b) providing a quantity of a compound having the formula: R₃R₂R₁Si-A-X (II), wherein X is a heterocycle, and dissolving (II) in an organic-based solution; and
c) mixing the solutions from steps a) and b) in the presence of a strong oxidizing agent for a sufficient period of time to allow them to react to form the formula I compound.

2. The process of Claim 1 wherein the strong acid is sulfuric acid, and the organic-based solution is a C₁-C₆ alcohol or benzyl alcohol.

3. The process of Claim 1 wherein R₁, R₂ and R₃ are methyl.

4. The process of Claim 1 wherein X is 1,3-dioxolane.

5. The process of Claim 1 wherein the metal hydrate is ferrous sulfate and the strong oxidizing agent is hydrogen peroxide.

## Patentansprüche

1. Verfahren zum Synthetisieren von Camptothecin-Derivaten mit der Formel: 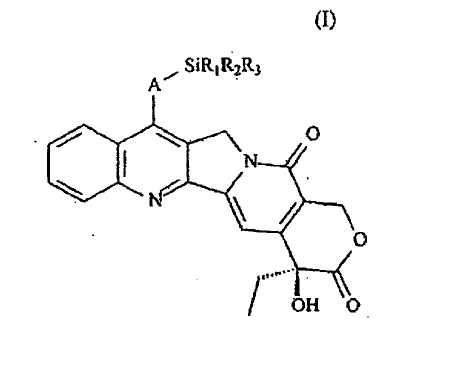 worin
A gleich -(CH₂)ₘ- ist;
m gleich 1 bis 6 ist; und
R₁, R₂ und R₃ jeweils C₁-C₆-Alkyl oder Aryl sind,
wobei das Verfahren folgendes aufweist:
a) Bereitstellen einer Menge von im wesentlichen reinem Camptothecin, Lösen des Camptothecins in einer Lösung einer starken Säure und Zugeben einer Menge eines Metallhydrat-Katalysators zu der Lösung der starken Säure;
b) Bereitstellen einer Menge einer Verbindung mit der Formel R₃R₂R₁Si-A-X (II), wobei X eine heterocyclische Einheit ist, und Lösen von (II) in einer Lösung auf organischer Basis; und
c) Mischen der Lösungen von den Schritten a) und b) in Gegenwart eines starken Oxidationsmittels für einen ausreichenden Zeitraum, damit sie reagieren können, so daß die Verbindung der Formel I entsteht.

2. Verfahren nach Anspruch 1, wobei die starke Säure Schwefelsäure ist und die Lösung auf organischer Basis ein C₁-C₆-Alkohol oder Benzylalkohol ist.

3. Verfahren nach Anspruch 1, wobei R₁, R₂ und R₃ Methyl sind.

4. Verfahren nach Anspruch 1, wobei X 1,3-Dioxolan ist.

5. Verfahren nach Anspruch 1, wobei das Metallhydrat Eisen(II)-sulfat ist und das starke Oxidationsmittel Wasserstoffperoxid ist.

## Revendications

1. Procédé de synthèse de dérivés de camptothécine de formule: 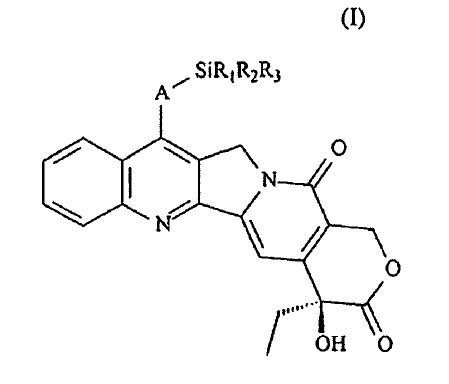 où A est -(CH₂)ₘ- ;
m vaut 1 à 6 ; et R₁, R₂ et R₃ sont individuellement un groupe alkyle en C₁ à C₆ ou aryle ; le procédé comprenant les étapes consistant à :
a) fournir une quantité de camptothécine sensiblement pure, dissoudre ladite camptothécine dans une solution d'un acide fort, et ajouter une quantité d'un catalyseur hydrate de métal à la solution d'acide fort ;
b) fournir une quantité d'un composé de formule: R₃R₂R₁Si-A-X (II), où X est un hétérocycle, et dissoudre (II) dans une solution à base organique ; et
c) mélanger les solutions des étapes a) et b) en présence d'un agent oxydant fort pendant une durée suffisante pour leur permettre de réagir afin de former le composé de formule (I).

2. Procédé selon la revendication 1, dans lequel l'acide fort est l'acide sulfurique, et la solution à base organique est un alcool en C₁ à C₆ ou l'alcool benzylique.

3. Procédé selon la revendication 1, dans lequel R₁, R₂ et R₃ sont un groupe méthyle.

4. Procédé selon la revendication 1, dans lequel X est un groupe 1,3-dioxolane.

5. Procédé selon la revendication 1, dans lequel l'hydrate de métal est le sulfate ferreux et l'agent oxydant fort est le peroxyde d'hydrogène.
